# EUROPEAN PATENT APPLICATION

(11) **EP 4 796 112 A1**
(43) Date of publication of application: **26.08.2026**
(21) Application number: 25158753.1
(22) Date of filing: 19.02.2025
(51) Int. Cl.: A61K 31/16, A61K 33/06, A61P 3/04

(54) **COMPOUND OR COMPOSITION COMPRISING THE SAME FOR USE IN THE TREATMENT OR IN THE PREVENTION OF OVERWEIGHTNESS AND OBESITY AND FOR WEIGHT MANAGEMENT AND/OR CONTROL**

(71) Applicant: Synapharm Industrial Synthesis, 4432 Ans (BE)
(72) Inventor: LEE, Edward, California, 91765 (US)
(74) Representative: Gevers Patents

(57) **Abstract**

The present invention relates to a compound or a composition comprising the same for use in the treatment or in the prevention of overweightness, of obesity and/or of the overweightness and the obesity associated metabolic disfunctions or disorders and/or of the overweightness and the obesity associated noncommunicable diseases (NCDs).

The present invention also relates to a use, in particular to a non-therapeutic use, of a compound or of a composition comprising the same, for managing / controlling weight, for controlling obesity, for controlling normal weight, for controlling overweightness, for supporting weight loss, for supporting weight maintenance, for suppressing food intake, for reducing appetite, for increasing lipolysis and/or for reducing / controlling weight gain.

## Description

### Field of the invention

The present invention generally relates to the field of weight management and/or control.

In particular, the present invention relates to the control / maintenance of normal weight, to the control of overweightness, to the control and to the treatment of obesity, to the treatment of the overweightness and the obesity associated metabolic disfunctions or disorders and/or to the treatment of the overweightness and the obesity associated noncommunicable diseases (NCDs) treatment.

More particularly, the present invention relates to a compound or a composition comprising the same for use in the treatment or in the prevention of overweightness, of obesity and/or of the overweightness and the obesity associated metabolic disfunctions or disorders and/or of the overweightness and the obesity associated noncommunicable diseases (NCDs).

The present invention also relates to a use, in particular to a non-therapeutic use, of a compound or of a composition comprising the same, for managing / controlling weight, for controlling obesity, for controlling normal weight, for controlling overweightness, for supporting weight loss, for supporting weight maintenance, for suppressing food intake, for reducing appetite, for increasing lipolysis and/or for reducing / controlling weight gain.

### Background of the invention

According to the World Health Organization (WHO), in 2022, 2.5 billion adults aged 18 years and older were overweight, including over 890 million adults who were living with obesity. This corresponds to 43% of adults aged 18 years and over (43% of men and 44% of women) who were overweight; an increase from 1990, when 25% of adults aged 18 years and over were overweight. In parallel, an estimated 37 million children under the age of 5 years were overweight.

Generally, overweight and obesity result from an imbalance of energy intake (diet) and energy expenditure (physical activity). In most cases obesity is a multifactorial disease due to obesogenic environments, psycho-social factors and genetic variants. In a subgroup of patients, single major etiological factors can be identified such as medications, diseases, immobilization, iatrogenic procedures, monogenic disease/genetic syndrome.

Overweight is a condition of excessive fat deposits while obesity is a chronic complex disease defined by excessive fat deposits that can impair health. Obesity can lead to increased risk of type 2 diabetes and heart disease, it can affect bone health and reproduction, it increases the risk of certain cancers and it influences the quality of living, such as sleeping or moving.

The diagnosis of overweight and obesity is made by measuring people's weight and height and by calculating the body mass index (BMI): weight (kg)/height² (m²). The body mass index is a surrogate marker of fatness and additional measurements, such as the waist circumference, can help the diagnosis of obesity. The BMI categories for defining obesity vary by age and gender in infants, children and adolescents.

For adults, WHO defines normal weight, overweight and obesity as follows:
- normal weight is a BMI between 18.5 and 24.9;
- overweight is a BMI greater than or equal to 25 (between 25 and 29.9); and
- obesity is a BMI greater than or equal to 30.

For children, age needs to be considered when defining overweight and obesity:
- for children under 5 years of age:
   - overweight is weight-for-height greater than 2 standard deviations above WHO Child Growth Standards median; and
   - obesity is weight-for-height greater than 3 standard deviations above the WHO Child Growth Standards median.
- for children aged between 5-19 years:
   - overweight is BMI-for-age greater than 1 standard deviation above the WHO Growth Reference median; and
   - obesity is greater than 2 standard deviations above the WHO Growth Reference median.

The health risks caused by overweight and obesity are well known. In 2019, higher-than-optimal BMI caused an estimated 5 million deaths from noncommunicable diseases (NCDs) such as cardiovascular diseases, diabetes, cancers, neurological disorders, chronic respiratory diseases, and digestive disorders (GBD 2019 Risk Factor Collaborators. "Global Burden of 87 Risk Factors in 204 Countries and Territories, 1990-2019: a systematic analysis for the global burden of disease study 2019". Lancet, 2020, 396:1223-1249). Being overweight in childhood and adolescence affects children's and adolescents' immediate health and is associated with greater risk and earlier onset of various NCDs, such as type 2 diabetes and cardiovascular diseases. Children with obesity are very likely to be adults with obesity and are also at a higher risk of developing NCDs in adulthood.

The economic impacts of the obesity epidemic are also important. If nothing is done, the global costs of overweight and obesity are predicted to reach US$ 3 trillion per year by 2030 and more than US$ 18 trillion by 2060 (Okunogbe et al., "Economic Impacts of Overweight and Obesity." 2nd Edition with Estimates for 161 Countries. World Obesity Federation, 2022).

Overweight and obesity, as well as their related noncommunicable diseases, are preventable and manageable. At the individual level, people may be able to reduce their risk by adopting preventive interventions at each step of the life cycle, starting from pre-conception and continuing during the early years. These include ensuring appropriate weight gain during pregnancy; practicing exclusive breastfeeding in the first 6 months after birth and continued breastfeeding until 24 months or beyond; supporting behaviors of children around healthy eating, physical activity, sedentary behaviors and sleep, regardless of current weight status; limiting screen time; limiting consumption of sugar sweetened beverages and energy-dense foods and promote other healthy eating behaviors; enjoying a healthy life (healthy diet, physical activity, sleep duration and quality, avoid tobacco and alcohol, emotional self-regulation); limiting energy intake from total fats and sugars and increase consumption of fruit and vegetables, as well as legumes, whole grains and nuts; and engaging in regular physical activity. At the same time, health practitioners need to assess the weight and height of people accessing the health facilities; provide counselling on healthy diet and lifestyles; when a diagnosis of obesity is established, provide integrated obesity prevention and management health services including on healthy diet, physical activity and medical and surgical measures; and monitor other NCD risk factors (blood glucose, lipids and blood pressure) and assess the presence of comorbidities and disability, including mental health disorders.

However, even if all these recommendations are followed, and even if follow-up is carried out with a health practitioner, the fact remains that overweight and obesity remain major issues with a major impact on the world's population in terms of health and cost to society, as indicated above.

Consequently, there is a real need for providing an effective compound and/or an effective composition comprising the same for use in the treatment or in the prevention of overweightness, of obesity and/or of the overweightness and the obesity associated metabolic disfunctions or disorders and/or of the overweightness and the obesity associated noncommunicable diseases (NCDs). There is also a real need for providing an effective compound and/or an effective composition comprising the same for a use, in particular for a non-therapeutic use, for managing / controlling weight, for controlling obesity, for controlling normal weight, for controlling overweightness, for supporting weight loss, for supporting weight maintenance, for suppressing food intake, for reducing appetite, for increasing lipolysis and/or for reducing / controlling weight gain.

### Summary of the invention

It is an object of the invention to provide an effective compound and/or an effective composition comprising the same for use in the treatment or in the prevention of overweightness, of obesity and/or of the overweightness and the obesity associated metabolic disfunctions or disorders and/or of the overweightness and the obesity associated noncommunicable diseases (NCDs).

Another object of the invention is to provide an effective compound and/or an effective composition comprising the same for a use, in particular for a non-therapeutic use, for managing / controlling weight, for controlling obesity, for controlling / maintaining normal weight, for controlling overweightness, for supporting weight loss, for supporting weight maintenance, for suppressing food intake, for reducing appetite, for increasing lipolysis and/or for reducing / controlling weight gain.

To this end, according to the invention, there is provided a compound (C) according to general Formula (I), or stereoisomer thereof, or a composition comprising the same, for use in the treatment or in the prevention of overweightness, of obesity and/or of the overweightness and the obesity associated metabolic disfunctions or disorders and/or of the overweightness and the obesity associated noncommunicable diseases (NCDs), 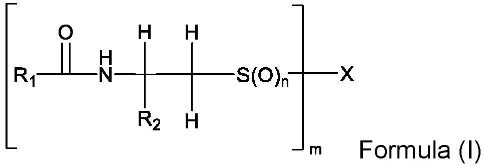 wherein:
- each of R₁ is a C₁₋₄ alkyl;
- each of R₂ is selected from H or COOH;
- n is an integer equal to 0, 2 or 3;
- m is an integer equal to 1 or 2; and
- X is selected from the H or an inorganic cation, wherein the inorganic cation is selected from an alkali metal cation or an alkaline earth metal cation.

In the present specification, the following terms have the following meanings:
"Body mass index" or "BMI" is the ratio of weight in kg divided by the height in metres, squared.
"Normal weight" is defined for an adult human as having a BMI between 18.5 and 24.9. In the context of the present invention, individuals presenting a normal weight and having a BMI between 18.5 and 24.9 are particularly targeted, in particular for controlling obesity, for controlling / maintaining normal weight, for controlling overweightness, for supporting weight loss, for supporting weight maintenance, for suppressing food intake, for reducing appetite, for increasing lipolysis and/or for reducing / controlling weight gain.
"Overweight" is defined for an adult human as having a BMI between 25 and 30 and is a condition of excessive fat deposits.
"Obesity" is a condition in which the natural energy reserve, stored in the fatty tissue of animals (including humans), is increased to a point where it is associated with certain health conditions or increased mortality. Obesity is recognized as being a disease defined by excessive fat deposits that can impair health. "Obese" is defined for an adult human as having a BMI greater than 30.
"Non communicable diseases (NCDs)" refers to a group of conditions that are not mainly caused by an acute infection, result in long-term health consequences and often create a need for long-term treatment and care. These conditions include cancers, cardiovascular disease, diabetes and chronic lung illnesses.
"Metabolic syndrome" refers to the co-occurrence of several known cardiovascular risk factors, including insulin resistance, obesity, atherogenic dyslipidemia and hypertension.
"Alkyl" has the broadest meaning generally understood in the art, and may include a moiety which is linear or branched, or a combination thereof.
"Alkyl" - alone or in combination refers to a straight or branched alkane-derived radical, for example, C_{F-G} alkyl defines a straight or branched alkyl radical having from F to G carbon atoms, e.g. C₁₋₄ alkyl defines a straight or branched alkyl radical having from 1 to 4 carbon atoms such as for example methyl, ethyl, 1-propyl, 2-propyl (isopropyl), 1-butyl, 2-butyl, 2-methyl-2-propyl (tert-butyl), 2-methyl-1-propyl (isobutyl).

According to the present invention, the compound (C) is according to general Formula (I) 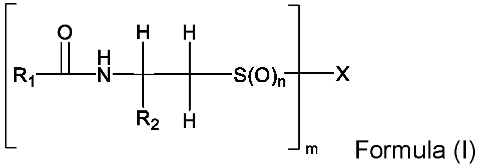 wherein:
- each of R₁ is a C₁₋₄ alkyl;
- each of R₂ is selected from H or COOH;
- n is an integer equal to 0, 2 or 3;
- m is an integer equal to 1 or 2; and
- X is selected from H or an inorganic cation, wherein the inorganic cation is selected from an alkali metal cation and an alkaline earth metal cation.

It will be understood that when n is equal to 0, S(O)ₙ represents a thiolate (-S-) moiety.

It will be further understood that when n is equal to 2, S(O)ₙ represents a sulfinate (-S(=O)O-) moiety.

It will be further understood that when n is equal to 3, S(O)ₙ represents a sulfonate (-S(=O)₂O-) moiety.

It will be understood that the nature of X, as detailed above, determines m.

In particular, provided that X of compound (C) of the present invention is H, m is equal to 1.

In particular, provided that X of compound (C) of the present invention is an alkali metal cation (i.e. a cation having a charge of +1, e.g. a lithium cation, a sodium cation or a potassium cation), m is equal to 1.

In particular, provided that X of compound (C) of the present invention is an alkaline earth metal cation (i.e. a cation having a charge of +2, e.g. a magnesium cation or a calcium cation), m is equal to 2.

Preferably, each of R₁ is a C₁₋₃ alkyl. More preferably, each of R₁ is selected from CH₃ or C₂H₅. Even more preferably each of R₁ is CH₃.

Preferably each of R₂ is H.

Preferably, X is an inorganic cation, wherein the inorganic cation is selected from the group consisting of an alkali metal cation and an alkaline earth metal cation. More preferably, X is an inorganic cation selected from the group consisting of a lithium cation, a sodium cation, a potassium cation, a magnesium cation and a calcium cation. More preferably X is selected from the group consisting of a sodium cation, a potassium cation, a magnesium cation and a calcium cation. More preferably, X is selected from a magnesium cation and a calcium cation. Even more preferably, X is a magnesium cation.

According to certain embodiments of the present invention, the compound (C) according to general Formula (I) is a compound of Formula (II) [compound (C) of class (I), herein after]: 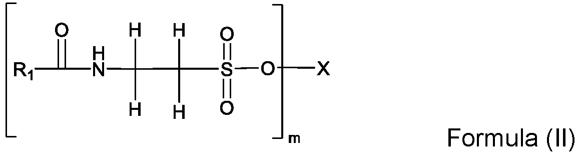 wherein R₁, m and X are as defined above for Formula (I).

According to a preferred embodiment of the present invention, compound (C) of class (I) is a compound of Formula (IIa): 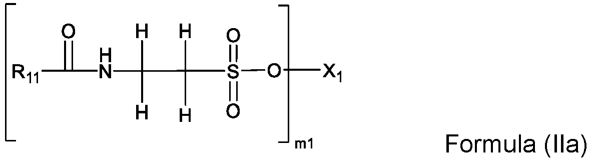 wherein:
- each of R₁₁ is a C₁₋₄ alkyl;
- m1 is an integer equal to 1 or 2;
- X₁ is an inorganic cation selected from the group consisting of an alkali metal cation and an alkaline earth metal cation;

Preferably, each of R₁₁ is a C₁₋₃ alkyl; more preferably, each of R₁₁ is selected from CH₃ or C₂H₅; even more preferably each of R₁₁ is CH₃.

Preferably, X₁ is an inorganic cation selected from the group consisting of a lithium cation, a sodium cation, a potassium cation, a magnesium cation and a calcium cation; more preferably X₁ is selected from the group consisting of a sodium cation, a potassium cation, a magnesium cation and a calcium cation; more preferably, X₁ is selected from a magnesium cation and a calcium cation; even more preferably, X₁ is a magnesium cation.

According to a preferred embodiment of the present invention, compound (C) of class (I) is a compound of Formula (IIb): 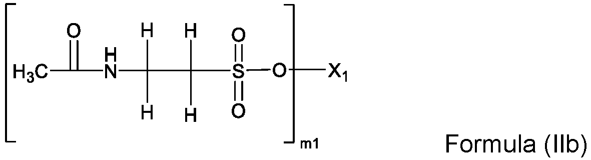 wherein m1 and X₁ have the same meaning as defined above for Formula (IIa).

According to a preferred embodiment of the present invention, compound (C) of class (I) is a compound of Formula (IIc):

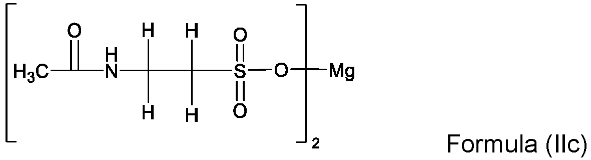

It will be understood that said compound according to Formula (IIc) is magnesium n-acetyl taurinate.

According to another embodiment of the present invention, the compound (C) according to general Formula (I) is a compound of Formula (III) [compound (C) of class (II), hereinafter]: 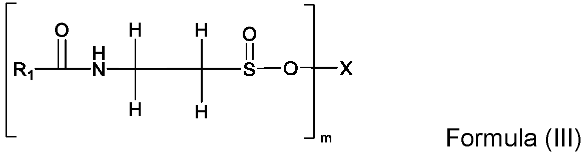 wherein R₁, m and X are as defined above for Formula (I).

According to a preferred embodiment of the present invention, compound (C) of class (II) is a compound of Formula (IIIa): 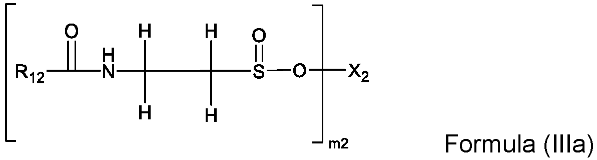 wherein:
- each of R₁₂ is a C₁₋₄ alkyl;
- m2 is an integer equal to 1 or 2;
- X₂ is an inorganic cation selected from the group consisting of an alkali metal cation and an alkaline earth metal cation;

Preferably, each of R₁₂ is a C₁₋₃ alkyl; more preferably, each of R₁₂ is selected from CH₃ or C₂H₅; even more preferably each of R₁₂ is CH₃.

Preferably, X₂ is an inorganic cation selected from the group consisting of a lithium cation, a sodium cation, a potassium cation, a magnesium cation and a calcium cation; more preferably X₂ is selected from the group consisting of a sodium cation, a potassium cation, a magnesium cation and a calcium cation; more preferably, X₂ is selected from a magnesium cation and a calcium cation; even more preferably, X₂ is a magnesium cation.

According to a preferred embodiment of the present invention, compound (C) of class (II) is a compound of Formula (IIIb): 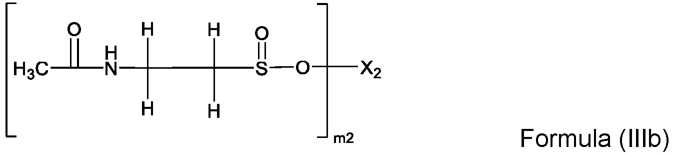 wherein m2 and X₂ have the same meaning as defined above for Formula (IIIa).

According to a preferred embodiment of the present invention, compound (C) of class (II) is a compound of Formula (IIIc):

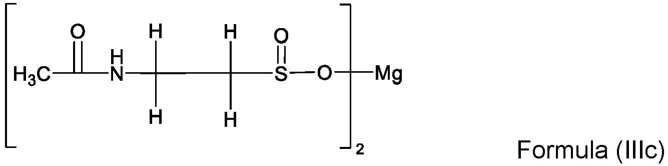

According to another embodiment of the present invention, the compound (C) according to general Formula (I) is a compound of Formula (IV) [compound (C) of class (III), hereinafter]: 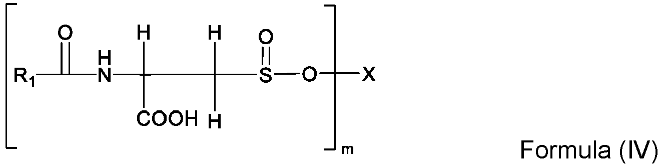 wherein R₁, m and X are as defined above for Formula (I).

According to a preferred embodiment of the present invention, compound (C) of class (III) is a compound of Formula (IVa): 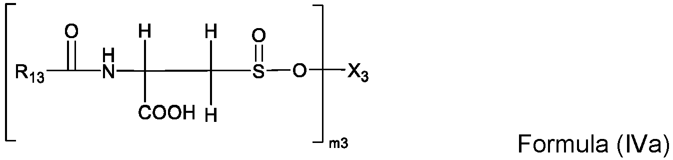 wherein:
- each of R₁₃ is a C₁₋₄ alkyl;
- m3 is an integer equal to 1 or 2;
- X₃ is an inorganic cation selected from the group consisting of an alkali metal cation and an alkaline earth metal cation;

Preferably, each of R₁₃ is a C₁₋₃ alkyl; more preferably, each of each of R₁₃ is selected from CH₃ or C₂H₅; even more preferably each of R₁₃ is CH₃.

Preferably, X₃ is an inorganic cation selected from the group consisting of a lithium cation, a sodium cation, a potassium cation, a magnesium cation and a calcium cation; more preferably X₃ is selected from the group consisting of a sodium cation, a potassium cation, a magnesium cation and a calcium cation; more preferably, X₃ is selected from a magnesium cation and a calcium cation; even more preferably, X₃ is a magnesium cation.

According to a preferred embodiment of the present invention, compound (C) of class (III) is a compound of Formula (IVb): 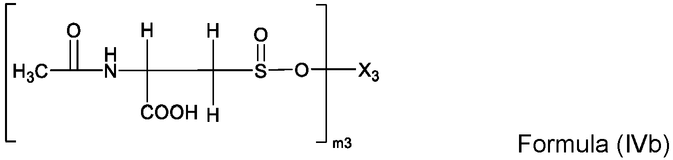 wherein m3 and X₃ have the same meaning as defined above for Formula (Va).

According to a preferred embodiment of the present invention, compound (C) of class (III) is a compound of Formula (IVc):

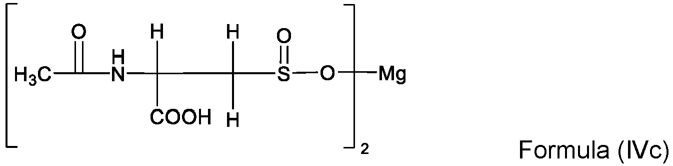

According to another embodiment of the present invention, the compound (C) according to general Formula (I) is a compound of Formula (V) [compound (C) of class (IV), herein after]: 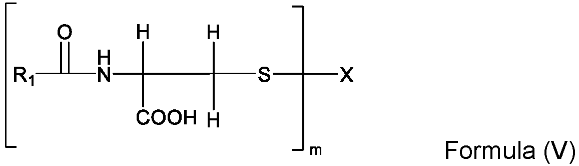 wherein R₁, m and X are as defined above for Formula (I).

According to a preferred embodiment of the present invention, compound (C) of class (IV) is a compound of Formula (Va): 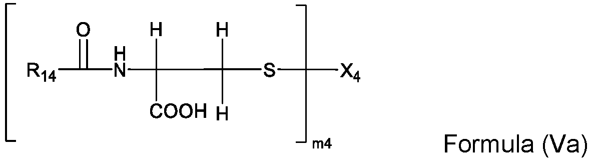 wherein:
- R₁₄ is a C₁₋₄ alkyl;
- m4 is an integer equal to 1 or 2;
- X₄ is an inorganic cation selected from the group consisting of an alkali metal cation and an alkaline earth metal cation.

Preferably, each of R₁₄ is a C₁₋₃ alkyl; more preferably, each of each of R₁₄ is selected from CH₃ or C₂H₅; even more preferably each of R₁₄ is CH₃.

Preferably, X₄ is an inorganic cation selected from the group consisting of a lithium cation, a sodium cation, a potassium cation, a magnesium cation and a calcium cation; more preferably X₄ is selected from the group consisting of a sodium cation, a potassium cation, a magnesium cation and a calcium cation; more preferably, X₄ is selected from a magnesium cation and a calcium cation; even more preferably, X₄ is a magnesium cation.

According to a preferred embodiment of the present invention, compound (C) of class (IV) is a compound of Formula (Vb): 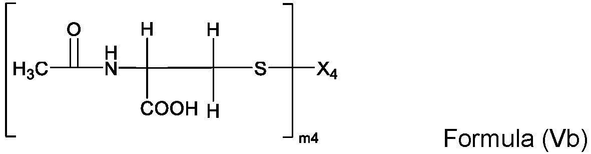 wherein m4 and X₄ have the same meaning as defined above for Formula (IVa).

According to a preferred embodiment of the present invention, compound (C) of class (IV) is a compound of Formula (Vc):

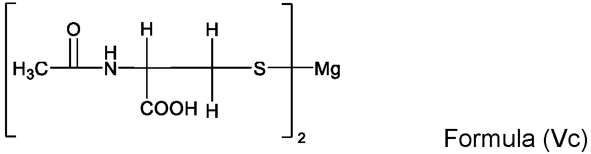

When any variable occurs more than one time in any constituent, each definition is independent. Whenever used hereinafter, the term compounds (C) of Formula (I)", or "the present compounds" or similar terms, it is meant to include all the compounds (C) of Formula (I), and stereochemically isomeric forms. One embodiment comprises the compounds (C) of Formula (I) or any subgroups of compounds of Formula (II), (IIa)-(IIc), (III), (IIIa)-(IIIc), (IV), (IVa)-(IVc), (V), (Va)-(Vc) specified herein, as well as the possible stereoisomeric forms thereof.

The compound (C) of Formula (I) as specified herein, or a compound of any of subgroups of compounds of Formula (II), (IIa)-(IIc), (III), (IIIa)-(IIIc), (IV), (IVa)-(IVc), (V), (Va)-(Vc) specified herein, have several centers of chirality and exist as stereochemically isomeric forms. The term "stereochemically isomeric forms" as used herein defines all the possible compounds made up of the same atoms bonded by the same sequence of bonds but having different three-dimensional structures which are not interchangeable, which the compound (C) of Formulae (I) as specified herein, or a compound of any of subgroups of compounds of Formula (II), (IIa)-(IIc), (III), (IIIa)-(IIIc), (IV), (IVa)-(IVc), (V), (Va)-(Vc) as specified herein, may possess.

Unless otherwise mentioned or indicated, the chemical designation of a compound (C) of Formulae (I) as specified herein, or a compound of any of subgroups of compounds of Formula (II), (IIa)-(IIc), (III), (IIIa)-(IIIc), (IV), (IVa)-(IVc), (V), (Va)-(Vc) as specified herein, encompasses the mixture of all possible stereochemically isomeric forms, which said compound may possess. Said mixture may contain all enantiomers of the basic molecular structure of said compound. All stereochemically isomeric forms of the compounds of the present invention both in pure form or mixed with each other are intended to be embraced within the scope of the present invention.

Pure stereoisomeric forms of compound (C) of Formulae (I) as specified herein, or a compound of any of subgroups of compounds of Formula (II), (IIa)-(IIc), (III), (IIIa)-(IIIc), (IV), (IVa)-(IVc), (V), (Va)-(Vc) as specified herein, and intermediates as mentioned herein are defined as isomers substantially free of other enantiomeric of the same basic molecular structure of said compounds or intermediates. In particular, the term "stereoisomerically pure" concerns compounds or intermediates having a stereoisomeric excess of at least 80% (i.e. minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and should be understood in a similar way, but then having regard to the enantiomeric excess of the mixture in question.

Pure stereoisomeric forms of the compounds and intermediates of this invention may be obtained by the application procedures known in the art.

A person skilled in the art is able to determine the absolute configuration of such compounds using art-known methods such as, for example, X-ray diffraction.

The present invention is also intended to include all isotopes of atoms occurring on the present to a compound (C) of compound (C) of Formulae (I) as specified herein, or a compound of any of subgroups of compounds of Formula (II), (IIa)-(IIc), (III), (IIIa)-(IIIc), (IV), (IVa)-(IVc), (V), (Va)-(Vc), as specified herein. Isotopes include those atoms having the same atomic number but different mass numbers. By way of general example and without limitation, isotopes of hydrogen include tritium and deuterium. Isotopes of carbon include C-13 and C-14.

In the context of the present invention, it has been determined that a compound (C) according to general Formula (I), or stereoisomer thereof, or a composition comprising the same has positive effects when used in the treatment or in the prevention of overweightness, of obesity and/or of the overweightness and the obesity associated metabolic disfunctions or disorders and/or of the overweightness and the obesity associated noncommunicable diseases (NCDs).

Preferably, according to the present invention, the associated metabolic disfunctions or disorders and/or associated noncommunicable diseases (NCDs) are selected from the group consisting of diabetes, hypertension, liver cirrhosis, metabolic syndrome, cardiovascular diseases, cancers, neurological disorders, chronic respiratory diseases, digestives disorders, and combinations thereof.

Advantageously, the compound or the composition comprising the same according to the present invention is a medicament, a food or a beverage product, a pet food product, a food additive or a nutraceutical or a dietary supplement.

Preferably, the compound or the composition comprising the same according to the present invention is intended for infants, children, and/or adults.

For example, the compound or the composition comprising the same according to the present invention is in an orally administrable form or in a enteral administrable form or in a parenteral administrable form, in particular in the form of a tablet, a pill, a capsule, a soluble powder, an oily solution, an effervescent tablet, a soft capsule, a solution including a beverage, a shot, or a in a medical food format.

Preferably, the composition according to the present invention may comprise protective hydrocolloids, excipients, diluents binders, film forming agents, flavouring agents, preservatives, stabilizers, buffers, lubricants, colorants, wetting agents, fillers, encapsulating agents, coatings, emulsifiers, surface active agents, solubilizing agents, adsorbents, carriers, dispersing agents, taste masking agents, gel forming agents, antioxidants and/or antimicrobials, and combinations thereof.

Advantageously, the composition according to the present invention may also comprise bioavailability enhancers, for example cyclodextrins, PEG, emulsions, micronized powder, liposomes, niosomes, phytosomes, exosomes, and combinations thereof.

Advantageously, the composition according to the present invention may also comprise minerals and micronutrients, for example calcium, magnesium, phosphorus, iron, zinc, copper, iodine, selenium, beta carotene, vitamin C, vitamin B1, vitamin B6, vitamin B2, niacin, vitamin B12, folic acid, biotin, 1 to 5 µg vitamin D and/or vitamin E, docosahexaenoic acid, eicosapentaenoic acid, and combinations thereof.

Advantageously, the composition according to the present invention may also comprise other natural ingredients for weight management support such as for example green tea extract, caffeine, glucomannan, chromium picolinate, chitosan, berberine, conjugated linoleic acid, prebiotic fibers (GOS, XOS, FOS, inulin, XOS), green kiwi fruit extract, yellow kiwi fruit extract, pineapple guava extract, cinnamon extract, and combinations thereof.

Another object of the present invention is a use, in particular a non-therapeutic use, of a compound (C) according to general Formula (I), or of stereoisomer thereof, or of a compound of any of the subgroups of compounds of Formula (II), (IIa)-(IIc), (III), (IIIa)-(IIIc), (IV), (IVa)-(IVc), (V), (Va)-(Vc), as specified herein, and according to any one of the embodiments presented hereinto, or of a composition comprising the same, for controlling obesity, for controlling / maintaining normal weight, for controlling overweightness, for supporting weight loss, for supporting weight maintenance, for suppressing food intake, for reducing appetite, for increasing lipolysis and/or for reducing / controlling weight gain.

In particular, the composition according to the present invention may be used to reduce weight gain or as a suppressor of food intake. More particularly, the composition according to the present invention may be used to treat or prevent overweightness and/or obesity resulting from a high-fat-diet.

Use, in particular non-therapeutic use, according to the invention, may consist in a dietary supplementation to support / to maintain a normal healthy weight and metabolism.

In the context of the present invention, it has been determined that a compound (C) according to general Formula (I), or stereoisomer thereof, or a composition comprising the same has positive effects when used, in particular when non-therapeutically used, for controlling obesity, for controlling / maintaining normal weight, for controlling overweightness, for supporting weight loss, for supporting weight maintenance, for suppressing food intake, for reducing appetite, for increasing lipolysis and/or for reducing / controlling weight gain.

Another object of the present invention is a method for (therapeutically) treating or preventing overweightness, obesity and/or the overweightness and the obesity associated metabolic disfunctions or disorders and/or the overweightness and the obesity associated noncommunicable diseases (NCDs) or for (non-therapeutically) managing / controlling weight, controlling obesity, controlling / maintaining normal weight, controlling overweightness, supporting weight loss, supporting weight maintenance, suppressing food intake, reducing appetite, increasing lipolysis and/or reducing / controlling weight gain, comprising the step of administering an effective amount of a compound according to the general Formula (I), or stereoisomer thereof, or a compound of any of the subgroups of compounds of Formula (II), (IIa)-(IIc), (III), (IIIa)-(IIIc), (IV), (IVa)-(IVc), (V), (Va)-(Vc), as specified herein, and according to any one of the embodiments presented hereinto, to an individual.

Advantageously, according to the method following the present invention, the associated metabolic disfunctions or disorders and/or associated noncommunicable diseases (NCDs) are selected from the group consisting of diabetes, hypertension, liver cirrhosis, metabolic syndrome, cardiovascular diseases, cancers, neurological disorders, chronic respiratory diseases, digestives disorders, and combinations thereof.

Preferably, according to the method following the present invention, the administration consists in the administration of a medicament, a food or beverage product, a pet food product, a food additive or a nutraceutical or a dietary supplement.

Preferably, according to the method following the present invention, the administration is intended for infants, children, and/or adults.

Advantageously, according to the method following the present invention, the administration is performed through an orally or a enteral administrable form or a parenteral administrable form, for example via a tablet, a pill, a capsule, a soluble powder, an oily solution, an effervescent tablet or a soft capsule, a solution including a beverage, a shot, or a medical food format.

As will be understood from the disclosures herein, the present invention also provides the use of a compound (C) of formula (I), or the particular compound of any of the subgroups of compounds of formula (II), (IIa)-(IIc), (III), (IIIa)-(IIIc), (IV), (IVa)-(IVc), (V), (Va)-(Vc), as specified herein, or compositions of the invention for the manufacture of a medicament, in particular a medicament for the treatment of overweightness, of obesity and/or of the overweightness and the obesity associated metabolic disfunctions or disorders and/or of the overweightness and the obesity associated noncommunicable diseases (NCDs).

To prepare the compositions of the present invention, an effective amount of the particular compound as the active ingredient is combined in intimate admixture with a carrier, in particular with a pharmaceutically acceptable carrier, which carrier may take a wide variety of forms depending on the form of preparation desired for administration. These compositions are desirable in unitary dosage form suitable, particularly, for administration orally, for enteral administration or for parenteral injection. For example, in preparing the compositions in oral dosage form, any of the usual media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. In the case of capsules, a compound (C) of formula (I), or the particular compound of any of the subgroups of compounds of formula (II), (IIa)-(IIc), (III), (IIIa)-(IIIc), (IV), (IVa)-(IVc), (V), (Va)-(Vc), may be present alone.

Because of their ease in administration, tablets and capsules represent the most advantageous oral dosage unit forms, in which case solid carriers may be employed. For enteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. All these forms of administration may also comprise bioavailability enhancers such as cyclodextrins, PEG, emulsions, micronized powder, liposomes, niosomes, phytosomes, exosomes, and combinations thereof.

It is especially advantageous to formulate the aforementioned compositions in unit dosage form for ease of administration and uniformity of dosage. Unit dosage form as used herein refers to physically discrete units suitable as unitary dosages, each unit containing a predetermined quantity of active ingredient calculated to produce the desired effect in association with the required pharmaceutical carrier. Examples of such unit dosage forms are tablets (including scored or coated tablets), capsules, pills, powder packets, wafers, injectable solutions or suspensions and the like, and segregated multiples thereof.

The compound, the compositions and the combinations of the present invention may be used as medicaments. Said use as a medicine or method of treatment comprises the systemic administration to subjects to be treated of an amount effective to combat the conditions. Consequently, the compound, the compositions and the combinations of the present invention can be used in the manufacture of a medicament useful for treating or preventing overweightness, obesity and/or the overweightness and the obesity associated metabolic disfunctions or disorders and/or the overweightness and the obesity associated noncommunicable diseases (NCDs) consisting notably of diabetes, hypertension, liver cirrhosis, metabolic syndrome, cardiovascular diseases, cancers, neurological disorders, chronic respiratory diseases, digestives disorders, and combinations thereof.

The compound, the compositions and the combinations of the present invention may be used as non-medicaments, for example as a food or a beverage product, a pet food product, a food additive or a nutraceutical or a dietary supplement.

The term "effective amount" as used herein means that amount of active compound that elicits the biological response in a tissue, system, animal or human that is being sought, in the light of the present invention, by a researcher, veterinarian, medical doctor or other clinician such as a nutritionist, which includes alleviation of the symptoms of the disease being treated.

The exact dosage and frequency of administration depends on the particular compound (C) of formula (I), or the particular compound of any of the subgroups of compounds of formula (II), (IIa)-(IIc), (III), (IIIa)-(IIIc), (IV), (IVa)-(IVc), (V), (Va)-(Vc), as specified herein, used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The present invention has been described in terms of specific embodiments, which are illustrative of the invention and not to be construed as limiting. More generally, it will be appreciated by persons skilled in the art that the present invention is not limited by what has been particularly shown and/or described hereinabove.

Use of the verbs "to comprise", "to include", "to be composed of", or any other variant, as well as their respective conjugations, does not exclude the presence of elements other than those stated.

Use of the article "a", "an" or "the" preceding an element does not exclude the presence of a plurality of such elements.

## Claims

1. A compound (C) according to general Formula (I), or stereoisomer thereof, or a composition comprising the same, for use in the treatment or in the prevention of overweightness, of obesity and/or of the overweightness and the obesity associated metabolic disfunctions or disorders and/or of the overweightness and the obesity associated noncommunicable diseases (NCDs), 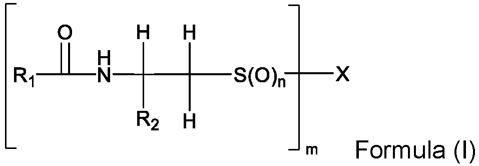 wherein:
- each of R₁ is a C₁₋₄ alkyl;
- each of R₂ is selected from H or COOH;
- n is an integer equal to 0, 2 or 3;
- m is an integer equal to 1 or 2;
- X is selected from H or an inorganic cation, wherein the inorganic cation is selected from the group consisting of an alkali metal cation and an alkaline earth metal cation.

2. The compound (C), or stereoisomer thereof, or the composition comprising the same, for use according to claim 1, wherein the associated metabolic disfunctions or disorders and/or associated noncommunicable diseases (NCDs) are selected from the group consisting of diabetes, hypertension, liver cirrhosis, metabolic syndrome, cardiovascular diseases, cancers, neurological disorders, chronic respiratory diseases, digestives disorders, and combinations thereof.

3. The compound (C), or stereoisomer thereof, or the composition comprising the same, for use according to claim 1 or 2, wherein the compound (C) is selected from those of Formula (IIa), (IIIa), (IVa) or (Va) hereinbelow: 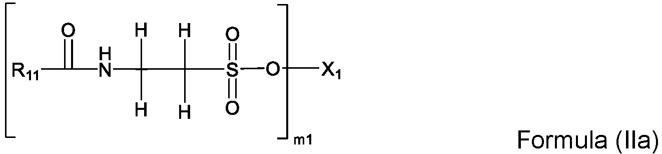 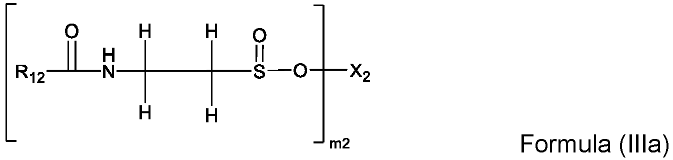 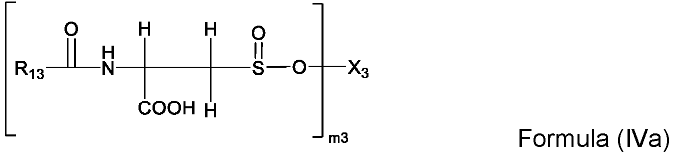 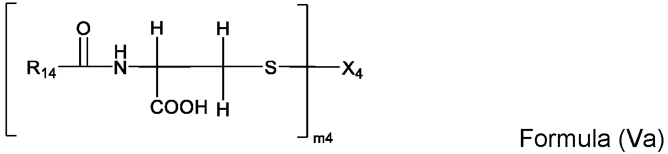 wherein:
- each of R₁₁ is a C₁₋₄ alkyl;
- m1 is an integer equal to 1 or 2;
- X₁ is an inorganic cation selected from the group consisting of an alkali metal cation and an alkaline earth metal cation;
- each of R₁₂ is a C₁₋₄ alkyl;
- m2 is an integer equal to 1 or 2;
- X₂ is an inorganic cation selected from the group consisting of an alkali metal cation and an alkaline earth metal cation;
- each of R₁₃ is a C₁₋₄ alkyl;
- m3 is an integer equal to 1 or 2;
- X₃ is an inorganic cation selected from the group consisting of an alkali metal cation and an alkaline earth metal cation;
- each of R₁₄ is a C₁₋₄ alkyl;
- m4 is an integer equal to 1 or 2;
- X₄ is an inorganic cation selected from the group consisting of an alkali metal cation and an alkaline earth metal cation.

4. The compound (C), or stereoisomer thereof, or the composition comprising the same, for use according to claim 1 or 2, wherein the compound (C) is selected from those of Formula (IIc), (IIIc), (IVc) or (Vc) hereinbelow:

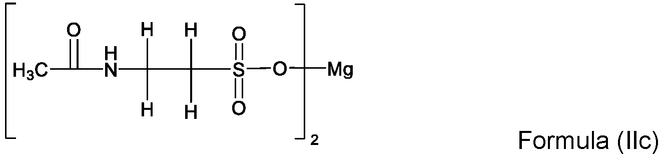

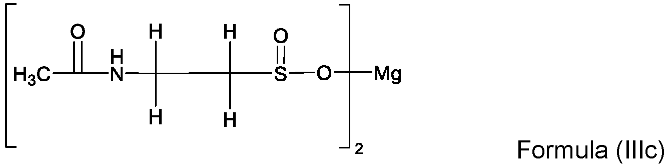

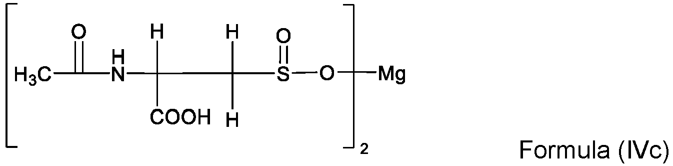

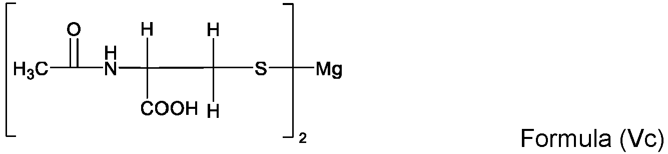

5. Use, in particular non-therapeutic use, of a compound (C) according to general Formula (I), or of stereoisomer thereof, or of a composition comprising the same, for managing / controlling weight, for controlling obesity, for controlling / maintaining normal weight, for controlling overweightness, for supporting weight loss, for supporting weight maintenance, for suppressing food intake, for reducing appetite, for increasing lipolysis and/or for reducing / controlling weight gain, 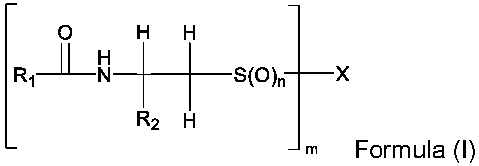 wherein:
- each of R₁ is a C₁₋₄ alkyl;
- each of R₂ is selected from H or COOH;
- n is an integer equal to 0, 2 or 3;
- m is an integer equal to 1 or 2;
- X is selected from H or an inorganic cation, wherein the inorganic cation is selected from the group consisting of an alkali metal cation and an alkaline earth metal cation.

6. Use, in particular non-therapeutic use, of a compound (C) according to general Formula (I), or of stereoisomer thereof, or of a composition comprising the same, according to claim 5, wherein the compound (C) is selected from those of Formula (IIa), (IIIa), (IVa) or (Va) hereinbelow: 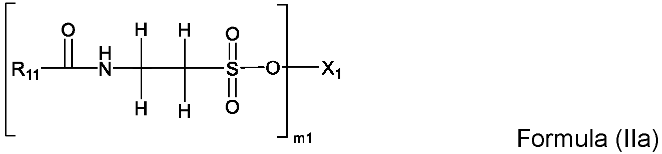 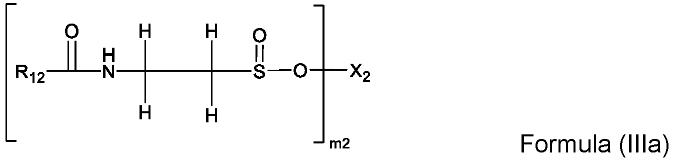 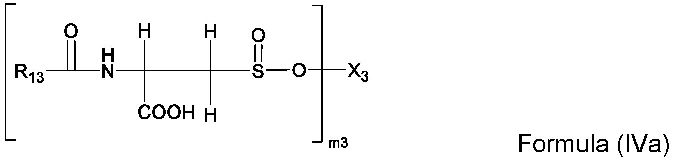 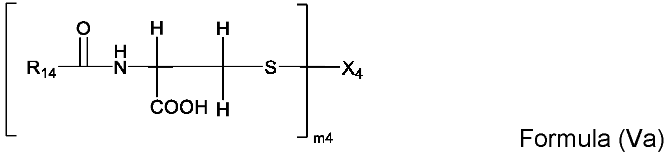 wherein:
- each of R₁₁ is a C₁₋₄ alkyl;
- m1 is an integer equal to 1 or 2;
- X₁ is an inorganic cation selected from the group consisting of an alkali metal cation and an alkaline earth metal cation;
- each of R₁₂ is a C₁₋₄ alkyl;
- m2 is an integer equal to 1 or 2;
- X₂ is an inorganic cation selected from the group consisting of an alkali metal cation and an alkaline earth metal cation;
- each of R₁₃ is a C₁₋₄ alkyl;
- m3 is an integer equal to 1 or 2;
- X₃ is an inorganic cation selected from the group consisting of an alkali metal cation and an alkaline earth metal cation;
- each of R₁₄ is a C₁₋₄ alkyl;
- m4 is an integer equal to 1 or 2;
- X₄ is an inorganic cation selected from the group consisting of an alkali metal cation and an alkaline earth metal cation.

7. Use, in particular non-therapeutic use, of a compound (C) according to general Formula (I), or of stereoisomer thereof, or of a composition comprising the same, according to claim 5, wherein the compound (C) is selected from those of Formula (IIc), (IIIc), (IVc) or (Vc) hereinbelow:

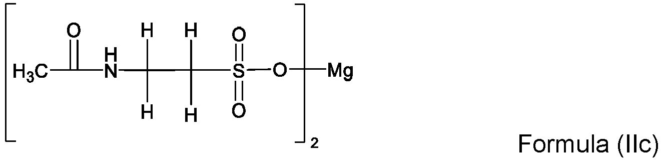

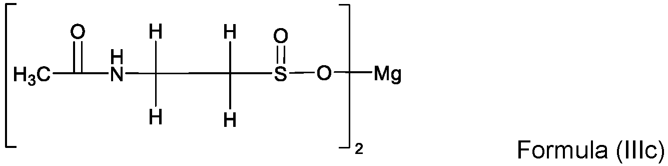

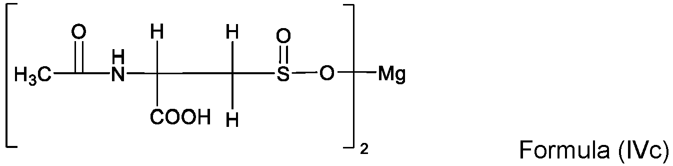

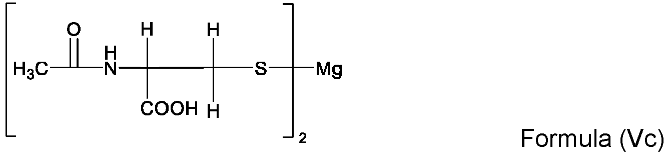

8. A method for treating or preventing overweightness, obesity and/or the overweightness and the obesity associated metabolic disfunctions or disorders and/or the overweightness and the obesity associated noncommunicable diseases (NCDs) or for managing / controlling weight, for controlling obesity, for controlling / maintaining normal weight, for controlling overweightness, for supporting weight loss, for supporting weight maintenance, for suppressing food intake, for reducing appetite, for increasing lipolysis and/or for reducing / controlling weight gain, comprising the step of administering an effective amount of a compound (C) according to general Formula (I), or stereoisomer thereof, to an individual: 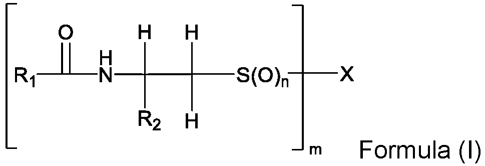 wherein:
- each of R₁ is a C₁₋₄ alkyl;
- each of R₂ is selected from H or COOH;
- n is an integer equal to 0, 2 or 3;
- m is an integer equal to 1 or 2;
- X is selected from H or an inorganic cation, wherein the inorganic cation is selected from the group consisting of an alkali metal cation and an alkaline earth metal cation.

9. The method according to claim 8, wherein the associated metabolic disfunctions or disorders and/or associated noncommunicable diseases (NCDs) are selected from the group consisting of diabetes, hypertension, liver cirrhosis, metabolic syndrome, cardiovascular diseases, cancers, neurological disorders, chronic respiratory diseases, digestives disorders, and combinations thereof.

10. The method according to claim 8 or 9, wherein the compound (C) according to general Formula (I), or stereoisomer thereof is selected from those of Formula (IIa), (IIIa), (IVa) or (Va) hereinbelow: 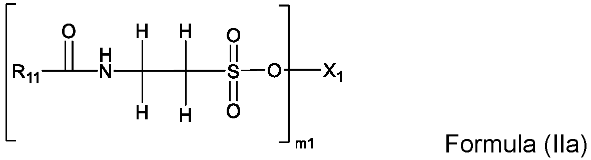 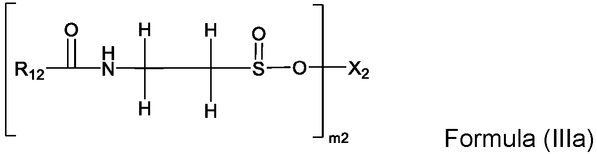 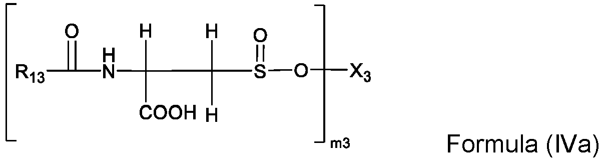 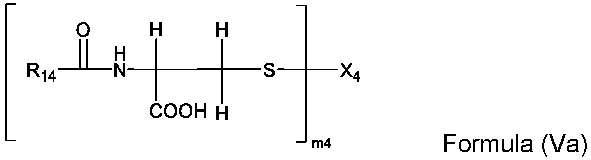 wherein:
- each of R₁₁ is a C₁₋₄ alkyl;
- m1 is an integer equal to 1 or 2;
- X₁ is an inorganic cation selected from the group consisting of an alkali metal cation and an alkaline earth metal cation;
- each of R₁₂ is a C₁₋₄ alkyl;
- m2 is an integer equal to 1 or 2;
- X₂ is an inorganic cation selected from the group consisting of an alkali metal cation and an alkaline earth metal cation;
- each of R₁₃ is a C₁₋₄ alkyl;
- m3 is an integer equal to 1 or 2;
- X₃ is an inorganic cation selected from the group consisting of an alkali metal cation and an alkaline earth metal cation;
- each of R₁₄ is a C₁₋₄ alkyl;
- m4 is an integer equal to 1 or 2
- X₄ is an inorganic cation selected from the group consisting of an alkali metal cation and an alkaline earth metal cation.

11. The method according to claim 8 or 9, wherein the compound (C) according to general Formula (I), or stereoisomer thereof, is selected from those of Formula (IIc), (IIIc), (IVc) or (Vc) hereinbelow:
